# EUROPEAN PATENT SPECIFICATION

(11) **EP 4 112 027 B1**
(45) Date of publication and mention of the grant of the patent: **21.02.2024**
(21) Application number: 22181621.8
(22) Date of filing: 28.06.2022
(51) Int. Cl.: A61F 13/15, A61F 13/494, A61F 13/496

(54) **LOWER BODY GARMENTS FOR WOMEN AND METHODS OF PREPARING SAME**
UNTERKÖRPER-BEKLEIDUNGSSTÜCKE FÜR FRAUEN UND VERFAHREN ZUM HERSTELLEN DAVON
VÊTEMENTS POUR LA PARTIE INFÉRIEURE DU CORPS POUR FEMMES ET LEURS PROCÉDÉS DE PRÉPARATION

(30) Priority: 28.06.2021 US 202117359943
(43) Date of publication of application: 04.01.2023
(73) Proprietor: Krupa, Jessica, West Palm Beach, FL 33407 (US)
(72) Inventor: Krupa, Jessica, West Palm Beach, FL 33407 (US)
(74) Representative: Basck Limited

(56) References cited:
- DE-A1-102008 057 840
- US-A1- 2010 249 736
- US-A1- 2018 014 983
- US-A1- 2021 030 605

## Description

### TECHNICAL FIELD

The aspects of the disclosed embodiments relate generally to undergarments and more specifically, lower body garments, comprising a gusset, for women.

### BACKGROUND

Every year, millions of women suffer from various gynaecological problems including, but not limited to, urinary tract infections (UTIs), vaginal infections including yeast and bacterial vaginosis (BV), vulvitis, itching, irritation and overall discomfort of the genitalia. Despite several medical advancements and educational programmes in this regard, the situation of female health and hygiene is failing to address this in the developed and the developing nations alike. While wearing panties to cover the external genitalia seems to be a solution, the problems arising from the use of panties itself have not been widely addressed.

Conventionally, available panties expose the external genitalia, especially vulva, to chemically treated synthetic fabrics found in panties. Notably, such synthetic fabrics may cause one or more of the aforementioned gynaecological problems. Moreover, the conventional panties lack enough breathability to prevent the growth of bacteria and yeast that cause most of the genital infections.

The undergarment industry introduced panty gussets as a solution to the breathability lacking in the conventional panties, by sewing fabric liners to the crotch portion of the panties to protect against synthetic materials used in the conventional panties. DE10200805840 discloses such a panty.

However, the available gussets don't cover the entire vulva, and expose the vulva to chemically treated synthetic materials such as polyester, nylon, lycra, spandex and other synthetic materials, thereby making the genitalia more prone to trapping heat, moisture and bacteria that can lead to infections. Moreover, use of products such as absorbent pads, disposable panty liners, and the like, over the conventional gusset could be a temporary solution. However, such disposable articles require frequent changing, as they can still lead to gynaecological issues. Moreover, while in use, such disposable articles expose the external genitalia to harmful chemicals and create an environment inside the undergarment which propagates yeast and bacterial infections. Furthermore, such disposable products are eventually dumped in a landfill thus creating another big challenge of waste management and environmental pollution.

Therefore, in light of the foregoing discussion, there exists a need to overcome the aforementioned drawbacks associated with existing approaches for improving feminine health and hygiene by using gussets that suitably cover the entire external genitalia, especially the vulva of the woman to prevent these issues.

### SUMMARY

The aspects of the disclosed embodiments are directed to a lower body garment for a woman and method of preparing the same. In one embodiment, the aspects of the disclosed embodiments seek to provide a solution to the existing problems of lower body garments that fail to provide a breathable space for the crotch portion of the female torso. An aim of the aspects of the disclosed embodiments is to provide a solution that overcomes at least partially the problems encountered in prior art, and provides a lower body garment comprising a gusset made from an organic, breathable, absorbent fabric that is configured to cover the vulva of the woman.

In one aspect, the aspects of the disclosed embodiments provide a lower body garment for a woman, the garment comprising
- a main body fabric having an inner side, an outer side opposite the inner side, a front end, a rear end opposite the front end, a symmetrical vertical axis having a length L1 between the front end and the rear end, and a pair of curved side ends each on either side of the symmetrical vertical axis having a width W1 therebetween; and
- a gusset fabric having an inner side, an outer side opposite the inner side, a front end, a rear end opposite the front end, a symmetrical vertical axis having a length L2 between the front end and the rear end, and a pair of curved side ends each on either side of the symmetrical vertical axis having a width W2 therebetween, the gusset fabric secured to the main body fabric such that:
   - the outer side of the gusset fabric is arranged to face the inner side of the main body fabric, wherein the front end, the rear end, and the pair of curved side ends of the gusset fabric corresponds to the main body fabric, and
   - the front end, the rear end, and the pair of curved side ends of the gusset fabric are secured to the main body fabric at seams of the gusset fabric,
wherein the inner side of the gusset fabric is operable to cover the vulva of the woman, when in use, and wherein the length L2 corresponds to a length of the vulva, as defined in claim 1.

In another aspect, the aspects of the disclosed embodiments provide a method of preparing a lower body garment for a woman, as defined in claim 6, the method comprising
- obtaining a main body fabric having an inner side, an outer side opposite the inner side, a front end, a rear end opposite the front end, a symmetrical vertical axis having a length L1 between the front end and the rear end, and a pair of curved side ends each on either side of the symmetrical vertical axis having a width W1 therebetween;
- obtaining a gusset fabric having an inner side, an outer side opposite the inner side, a front end, a rear end opposite the front end, and a symmetrical vertical axis having a length L2 between the front end and the rear end, and a pair of curved side ends each on either side of the symmetrical vertical axis having a width W2 therebetween; and
- securing the gusset fabric to the main body fabric such that:
- the outer side of the gusset fabric is arranged to face the inner side of the main body fabric, wherein the front end, the rear end, and the pair of curved side ends of the gusset fabric correspond to the main body fabric, and
- the front end, the rear end, and the pair of curved side ends of the gusset fabric are secured to the main body fabric at seams of the gusset fabric,
wherein the inner side of the gusset fabric is operable to cover the vulva of the woman, when in use, and wherein the length L2 corresponds to a length of the vulva.

The aspects of the disclosed embodiments substantially eliminate or at least partially address the aforementioned problems in the prior art, and enable a robust lower body garment that provides a better coverage of the external genitalia, allows space for the crotch portion of the female torso and enhances female health and hygiene. Additionally, beneficially, the gusset fabric is secured to a main body fabric of the lower body garment to provide a durable and robust product. Moreover, the effective securing of the gusset fabric to the main body fabric throughout the edges of the gusset fabric ensures that the gusset fabric does not move, shift or roll during use or washing, for example, thereby providing for a reusable product.

Additional aspects, advantages, features and objects of the aspects of the disclosed embodiments would be made apparent from the drawings and the detailed description of the illustrative embodiments construed in conjunction with the appended claims that follow.

It will be appreciated that features of the aspects of the disclosed embodiments are susceptible to being combined in various combinations without departing from the scope of the aspects of the disclosed embodiments as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the aspects of the disclosed embodiments, exemplary constructions of the disclosure are shown in the drawings. However, the aspects of the disclosed embodiments are not limited to specific methods and instrumentalities disclosed herein. Moreover, those skilled in the art will understand that the drawings are not to scale. Wherever possible, like elements have been indicated by identical numbers.

The aspects of the disclosed embodiments will now be described, by way of example only, with reference to the following diagrams wherein:
FIGs. 1A, 1B, 1C, and 1D are perspective views of a lower body garment, implemented as a low-rise hipster, for a woman, in accordance with the aspects of the disclosed embodiments;
FIGs. 2A and 2B are perspective views of a lower body garment, implemented as a high-rise hipster, for a woman, in accordance with the aspects of the disclosed embodiments;
FIGs. 3A and 3B are perspective views of a lower body garment, implemented as a mid-rise bikini, for a woman, in accordance with the aspects of the disclosed embodiments;
FIGs. 4A and 4B are perspective views of a lower body garment, implemented as a high-rise bikini, for a woman, in accordance with the aspects of the disclosed embodiments;
FIGs. 5A and 5B are perspective views of a lower body garment, implemented as a low-rise thong, for a woman, in accordance with the aspects of the disclosed embodiments;
FIGs. 6A. and 6B are perspective views of a lower body garment, implemented as a high-rise thong, for a woman, in accordance with the aspects of the disclosed embodiments; and
FIG. 7 is a flowchart of steps of a method of preparing a lower body garment for a woman, in accordance with an aspect of the disclosed embodiments.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

### DESCRIPTION (Clean Copy)

The following detailed description illustrates exemplary aspects of the disclosed embodiments and ways in which they can be implemented. Although some modes of carrying out the aspects of the disclosed embodiments have been disclosed, those skilled in the art would recognize that other embodiments for carrying out or practising the aspects of the disclosed embodiments are also possible.

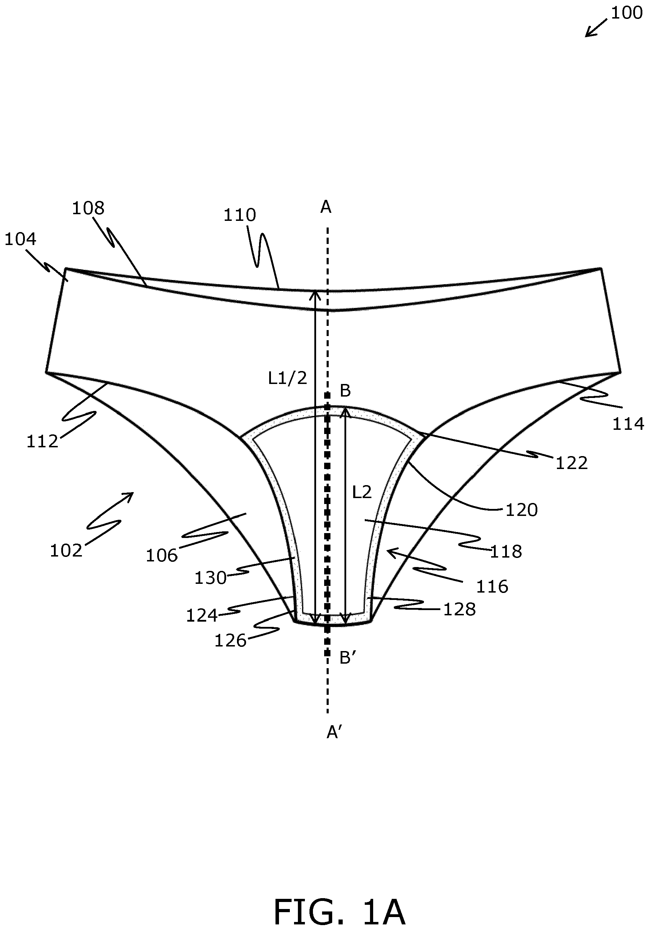

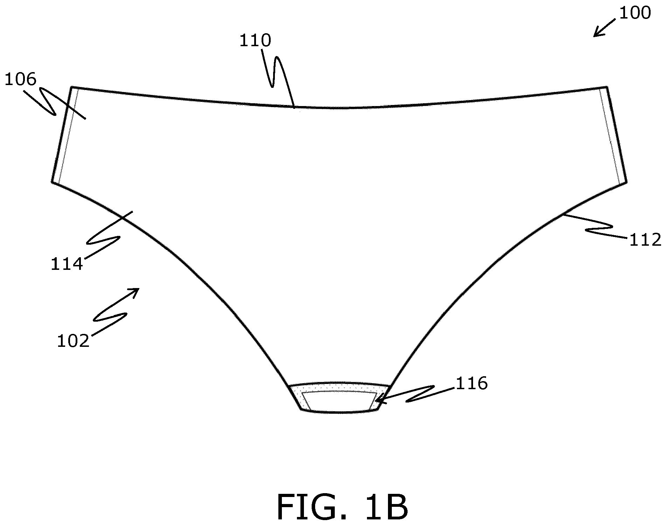

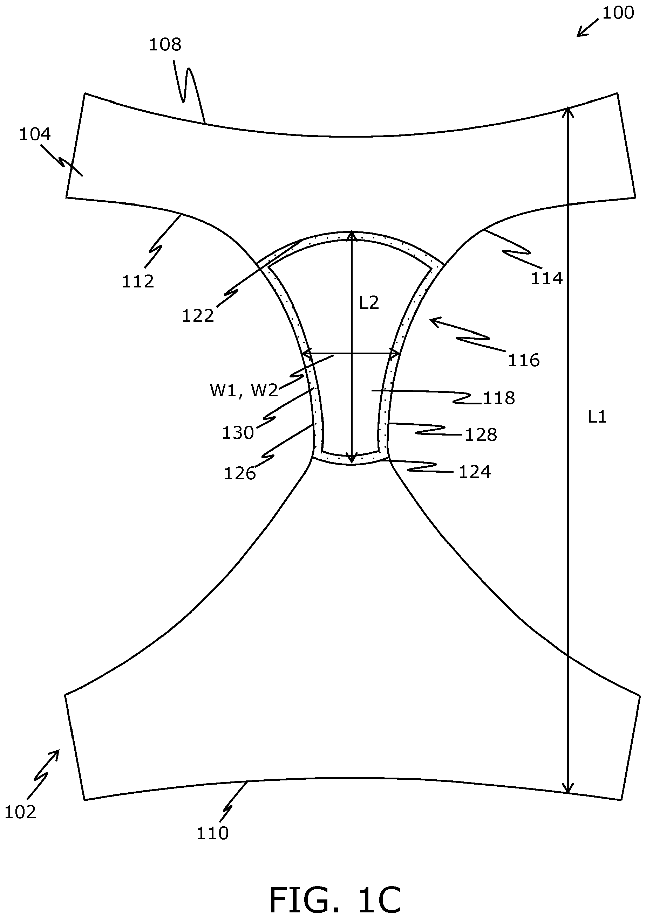

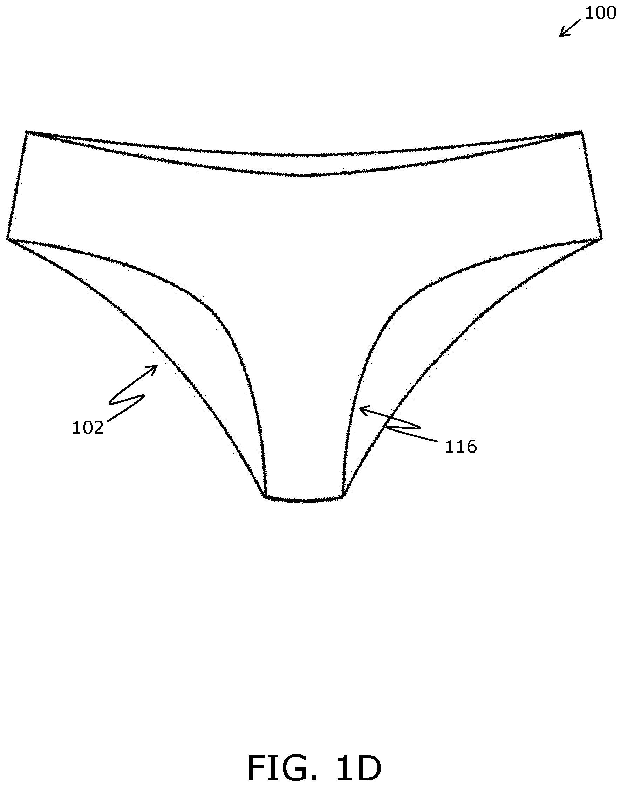

Referring to FIGs. 1A, 1B, 1C, and 1D, illustrated are perspective views of a lower body garment **100,** implemented as a low-rise hipster, for a woman, in accordance with an aspect of the disclosed embodiments. As shown, FIG.1A depicts a front view of the lower body garment **100** of an inside surface thereof, FIG. 1B depicts a rear view of the lower body garment **100** of an inside surface thereof, FIG. 1C depicts an inner surface of the lower body garment **100,** and FIG. 1D depicts a front view of the lower body garment **100** from an outside surface thereof. Beneficially, the lower body garment **100** is a robust, durable and reusable product that enables maintaining female health and hygiene by completely covering the vulva of the female torso.

Throughout the present disclosure, the term *"lower body garment"* **100** as used herein refers to a close-fitting (namely, form-fitting) garment worn usually next to skin around the pelvic region (between abdomen and thighs) of a human torso, especially that of a woman. The lower body garment **100** typically comprises at least a waistband that runs along the waist of the woman, a crotch panel to cover at least partially external genitals of the woman, and a pair of leg openings. Optionally, the external genitals include a vulva. The vulva typically includes a clitoris, a labia majora, a labia minora, a vestibule, a urethral meatus, an introitus and a perineum. Optionally, the lower body garment **100** could have different forms and sizes to suit different body-types and purposes. Optionally, the lower body garment **100** includes various panty styles, such as a boyshort, a hipster, a thong, a bikini, a G-string, a tanga, and the like, in various rises including a low-rise, a mid-rise, and a high-rise. Moreover, optionally, the lower body garment **100** could be used in other lower body garments, such as a shapewear, activewear, and everyday wear such as leggings, pantyhose, stockings, knickers, underpants, and the like. It will be appreciated that the lower body garment **100** may be available in different sizes ranging from women's extra-small (XXS and XS) to extra-large (XL), extra extra-large (XXL), and women's plus sizes from 1X to 5X. In an example, the size of the lower body garment **100** may be any of: XXS, XS, Small (S), Medium (M), large (L), extra-large (XL), extra extra-large (XXL), 1X, 2X, 3X, 4X and 5X, to suit women of different sizes.

The lower body garment **100** comprises a main body fabric **102** having an inner side **104,** an outer side **106** opposite the inner side **104,** a front end **108,** a rear end **110** opposite the front end **108,** a symmetrical vertical axis **A-A'** having a length **L1** between the front end **108** and the rear end **110,** and a pair of curved side ends **112, 114** each on either side of the symmetrical vertical axis **A-A'** having a width **W1** therebetween. Throughout the present disclosure, the term *"main body fabric"* **102** as used herein refers to a primary piece of fabric (namely a woven, non-woven or knitted cloth) that forms the base material of the lower body garment **100.** Typically, the main body fabric **102** forms the lower body garment **100.** In this regard, the main body fabric **102** is cut and stitched and/or bonded to result in the waistband (namely, a portion of the main body fabric **102** that encircles the waist of the wearer, i.e. the woman), the crotch panel (namely, a portion of the main body fabric **102** that covers external genitalia of the wearer, i.e. the woman), and the pair of leg openings (namely, parallel openings in the main body fabric **102** that allow arranging the crotch panel of the main body fabric **102** in between the legs of the wearer, i.e. the woman) of the lower body garment **100.** It will be appreciated that, when in use, the inner side **104** lies next to the skin of the torso and is expected to be a skin-friendly fabric, while the outer side **106** contributes mainly to the aesthetics of the lower body garment **100.** Moreover, the front end **108** and rear end **110** correspond respectively to a front anatomical structure and a rear anatomical structure of the user, that is the woman using the said lower body garment **100.** Moreover, the lower body garment **100** comprises the crotch panel at a pre-defined distance from the waistband of the lower body garment **100** extending from at least a part of the front end **108** up to a part of the rear end **108.** It will be appreciated that the main body garment **102** is vertically symmetrical along the entire length of the main body garment **102.**

Optionally, the main body fabric **102** is selected from a group comprising: organic cotton, cotton, cotton spandex, bamboo, bamboo spandex, hemp, linen, muslin, synthetic, wool, wool blend, elastane, rayon, nylon, satin, silk, lace, organza, jersey, net, charmeuse, lycra, spandex, polyester, micro modal, merino wool, or any combination thereof. It will be appreciated that the main body fabric **102** is selected to provide the lower body garment **100** with the desired characteristics, such as for example both aesthetic and comfort.

Moreover, the garment **100** comprises a gusset fabric **116** having an inner side **118,** an outer side **120** opposite the inner side **118,** a front end **122,** a rear end **124** opposite the front end **122,** a symmetrical vertical axis **B-B'** having a length **L2** between the front end **122** and the rear end **124,** and a pair of curved side ends **126, 128** each on either side of the symmetrical vertical axis **B-B'** having a width **W2** therebetween. Throughout the present disclosure, the term *"gusset fabric"* **116** as used herein refers to a secondary (relatively smaller as compared to the main body fabric **102**) piece of fabric (namely a woven, non-woven or knitted cloth) that is arranged to line the crotch panel of the lower body garment **100.** Optionally, the gusset fabric **116** is a single layer fabric made of a single type (or length) of fabric. The gusset fabric **116** is expected to be a skin-friendly, breathable and absorbent fabric that is optimum for feminine health and hygiene. Beneficially, using a fabric woven from organic cotton fibres that are breathable and absorbent that prevent growth of bacteria and yeast in the dark moist environments of the female intimate areas that are the leading causes of majority of urinary tract infections, bacterial infections, vulvitis, smells, odours, itching, irritation, and an overall misery of the genitalia. Additionally, beneficially, gusset fabric **116** made from organic, breathable, absorbent fibres promote female health and hygiene.

Optionally, the gusset fabric **116** is selected from a group comprising: organic cotton, cotton, linen, muslin, bamboo, hemp, wool, wool blend, elastane, or any combination thereof. It will be appreciated that the aforesaid fabrics for making the gusset fabric **116** are made of organic and breathable fibres with a high absorption quality. Optionally, the gusset fabric **116** is made from a 100% organic cotton fibre. Moreover, the gusset fabric **116** may be made using any fabric combination that is optimum for feminine health and hygiene. Moreover, the gusset fabric **116** may be selected from fabrics that are not chemically treated (namely, chemical-free fabrics), and therefore suitable for preventing gynaecological problems.

Furthermore, the gusset fabric **116** is secured to the main body fabric **102** such that the outer side **120** of the gusset fabric **116** is arranged to face the inner side **104** of the main body fabric **102,** wherein the front end **122,** the rear end **124,** and the pair of curved side ends **126, 128** of the gusset fabric **116** corresponds to the main body fabric **102.** It will be appreciated that the term *"correspond"* as used herein refers to an arrangement of the gusset fabric **116** and the main body fabric **102** such that the inner side **118** and the outer side **120** of the gusset fabric **116** are parallel to the inner side **104** and the outer side **106** of the main body fabric **102,** the front end **122** of the gusset fabric **116** is parallel to the front end **108** of the main body fabric **102,** the rear end **124** of the gusset fabric **116** is parallel to the rear end **110** of the main body fabric **102,** the symmetrical vertical axis **B-B'** of the gusset fabric **116** is parallel to the symmetrical vertical axis **A-A'** of the main body fabric **102,** and the pair of curved side ends **126, 128** of the gusset fabric **116** is parallel to the pair of curved side ends **112, 114** of the main body fabric **102.** It will be appreciated that the term *"correspond"* does not imply that the aforesaid sides or ends coincide, i.e. the front end **122** of the gusset fabric **116** with the front end **108** of the main body fabric **102,** the rear end **124** of the gusset fabric **116** with the rear end **110** of the main body fabric **102,** and the curved side ends **126, 128** of the gusset fabric **116** with the curved side ends **112, 114** of the main body fabric **102**.

Moreover, the pair of curved side ends **126, 128** of the gusset fabric 116 are secured to the main body fabric **102** at seams **130** of the gusset fabric **116.** The term *"seam"* **130** as used herein refers to edges of the gusset fabric **116** corresponding to the front end **122,** the rear end **124,** and the pair of curved side ends **126, 128.** Notably, the seams **130** of the gusset fabric **116** are equal throughout the gusset fabric **116.** Alternatively, the width of seams **130** may vary on the front end **122,** the rear end **124,** and/or the pair of curved side ends **126, 128,** based on the grading of the lower body garment size. Securing is typically performed at the seams **130.**

Throughout the present disclosure, the term *"secured"* as used herein refers to attaching firmly in a manner to prevent the attached parts from moving against each other.

The gusset fabric **116** is secured to the main body fabric **102** along at least one of: the front end **122** of the gusset fabric **116,** the rear end **124** of the gusset fabric **116,** the pair of curved side ends **126, 128** of the gusset fabric **116,** or a combination thereof. Preferably, the gusset fabric **116** is secured to the main body fabric **116** along all of ends of the gusset fabric **116,** i.e. the front end **122,** the rear end **124,** the pair of curved side ends **126, 128,** to enable a robust securing of the gusset fabric **116** to the main body fabric **116.** Beneficially, securing the gusset fabric **116** at all its four ends to the main body fabric **102** prevents the gusset fabric **116** from rolling or displacing during use, or moving or shifting during wearing or while washing. Additionally, beneficially, the sideways securing of the gusset fabric **116** and the main body fabric **102** enhances the comfort level associated with the gusset fabric **116.** However, alternatively, securing could be performed throughout the gusset fabric **116** and the main body fabric **102,** i.e. besides at all its four ends thereof, the gusset fabric **116** is secured to the main body fabric **102** at its middle portion also, i.e. portion covered between the pair of curved side ends **126, 128** and the front end **122** and the rear end **124.** It will be appreciated that securing of the gusset fabric **116** to the main body fabric **102** as mentioned above ensures a durable final product. Moreover, securing of the gusset fabric **116** to the main body fabric **102** as mentioned above ensures only the skin-friendly fabric, i.e. the gusset fabric **116,** to be next to the vulva of the woman. Furthermore, securing the gusset fabric **116** to the main body fabric **102** as mentioned above results in a more secure lower body garment **100,** providing a more comfortable feel and a better fit experience by the woman.

Optionally, the gusset fabric **116** is secured to the main body fabric **102** using at least one of: a stitch, a bond, an adhesive. Optionally, the means for securing is selected based on the types of gusset fabric **116** and the main body fabric **102.** The term *"stitch"* typically refers to joining the gusset fabric **116** and the main body fabric **102** using a thread and needle (by hands or a sewing machine), for example. The term *"bond"* typically relates to a stitch-less joining (namely, fusing, gluing or adhering) of the gusset fabric **116** and the main body fabric **102** using heat, ultrasonic waves, for example. Typically, securing using bond is suitable for thermoplastic fabrics, however, bonding, using an additional adhesive in between the two layers of fabric, such as the gusset fabric **116** and the main body fabric **102,** could be used universally for all types of fabrics. The term *"adhesive"* refers to a fabric-specific glue and/or tape that is used between two layers of fabric, i.e. the gusset fabric **116** and the main body fabric **102,** and set by application of pressure thereon. Beneficially, the securing of the gusset fabric **116** to the main body fabric **102** does not result in a visible impression on the outer surface of the lower body garment **100.**

Moreover, the inner side **118** of the gusset fabric **116** is operable to cover vulva (not shown) of the woman, when in use, and wherein the length **L2** corresponds to a length of the vulva. Optionally, when in use, the gusset fabric **116** is operable to cover vulva with the front end **122** ending at the mons pubis. The length **L2** of the gusset fabric **116** increases corresponding to the size of the lower body garment **100** in order to provide a fuller coverage to the vulva. It will be appreciated that the length **L2** of the gusset fabric **116** could be of different measurements in order to fit on the lower body garment **116** ranging typically from XXS, XS, S, M, L, XL, XXL, 1X 2X, 3X, 4X, 5X, and all other women's sizes. Moreover, the width **W2** of the gusset fabric **116** could be of different measurements in order to fit on the lower body garment **116** ranging typically from XXS, XS, S, M, L, XL, XXL, 1X 2X, 3X, 4X, 5X, and all other women's sizes.

The length **L2** is smaller than the length **L1** and is at least 5 inches (1 inch = 2,54 cm) and curved at the front end **122** of the gusset fabric **116** to cover the vulva. Typically, the length **L2** is in a range from 5 to 12 inches. The length **L2** may for example be from 5, 6, 7, 8, 9, 10 or 11 inches up to 6, 7, 8, 9, 10, 11 or 12 inches. It will be appreciated that the above-mentioned range may include a range of sizes such as 5¼ , 6½, 7¾, 8¼, 10 ½, and the like. Beneficially, the curved shape of the front end **122** of the gusset fabric **116** enables complete protection of the vulva.

Optionally, the gusset fabric is bonded on the inner side thereof that covers the vulva. The inner side **118** of the gusset fabric **116** ensures proper comfort to the user, when the lower body garment **100** is worn by the user .

The width **W2** could be marginally smaller or larger, by ±0.05-0.15 inch than the width **W1,** however, the curved ends **126, 128** of the gusset fabric **116** and the pair of curved ends **112, 114** are secured together. It will be appreciated that the marginally smaller width **W2** provides 20 a tolerance limit from the edge to edge of the seams of the gusset fabric **116** and the main body fabric **102.** In an example, a tolerance could be of 0.15 inch (or ⅛ inch).

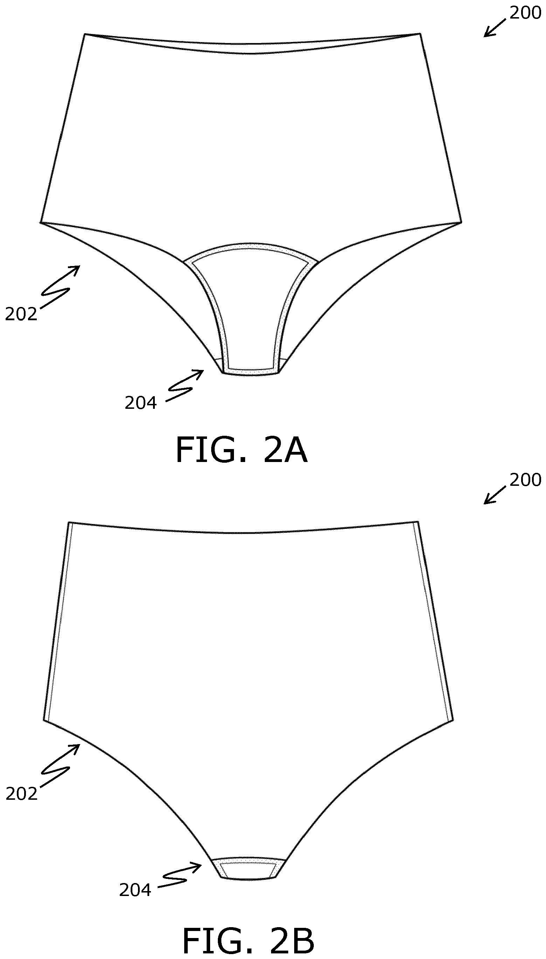

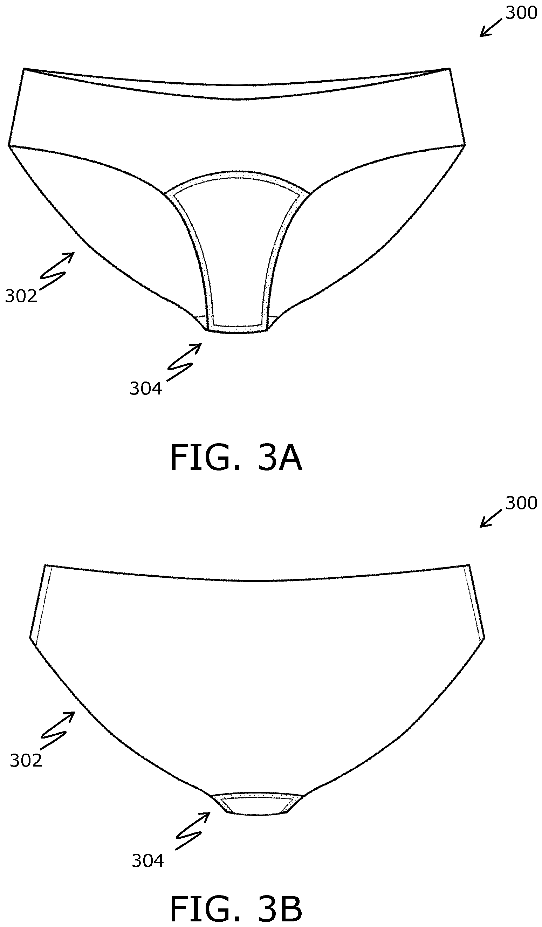

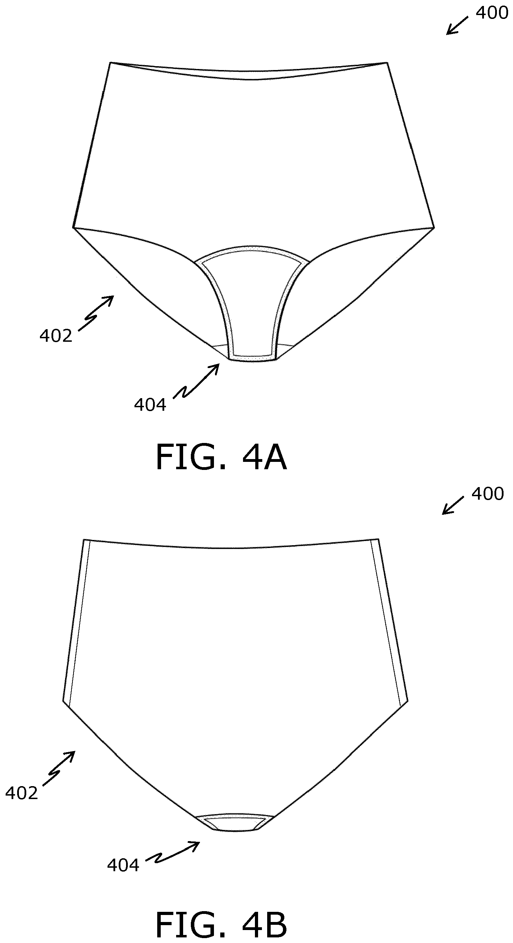

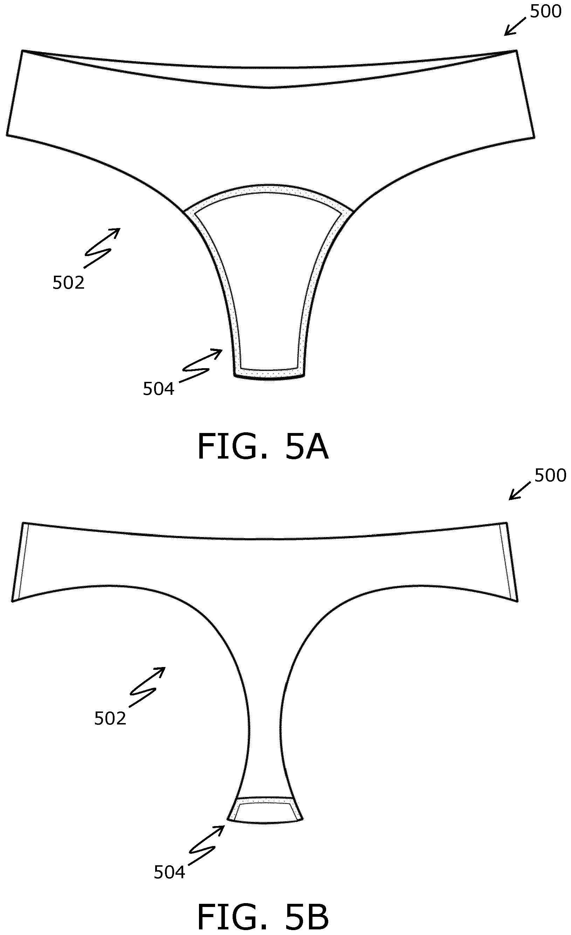

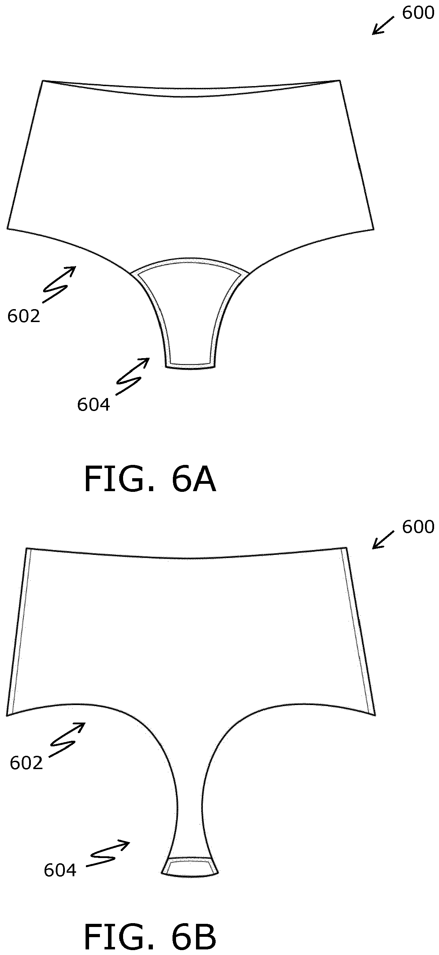

Referring to FIGs. 2A, 2B, 3A, 3B, 4A, 4B, 5A, 5B, 6A and 6B, illustrated are perspective views of different styles of a lower body garment, i.e. **200**, **300**, **400**, **500** and **600**, for a woman, in accordance with various aspect of the disclosed embodiments. As shown, FIGs. 2A, 3A, 4A, 5A and 6A depict a front view of an inside surface of the lower body garment, i.e. **200**, **300**, **400**, **500** and **600**, respectively, and the FIGs. 2B, 3B, 4B, 5B and 6B depict the back view of an inside surface of the lower body garment, i.e. **200**, **300**, **400**, **500** and **600.** Moreover, the lower body garment is implemented as a high-rise hipster **200**, a mid-rise bikini **300**, a high-rise bikini **400**, a low-rise thong **500** and a high-rise thong **600.** It will be appreciated that the lower body garment, i.e. **200**, **300**, **400**, **500** and **600** are similar in embodiment to the lower body garment **100** of FIG. 1. Each of the lower body garment, i.e. **200**, **300**, **400**, **500** and **600**, comprises a main body fabric **202, 302**, **402**, **502**, **602** and the gusset fabric **204**, **304**, **404**, **504**, **604**, the gusset fabric **204**, **304**, **404**, **504**, **604** secured to the main body fabric **202**, **302**, **402**, **502**, **602** at seams of the gusset fabric **204**, **304**, **404**, **504**, **604.**

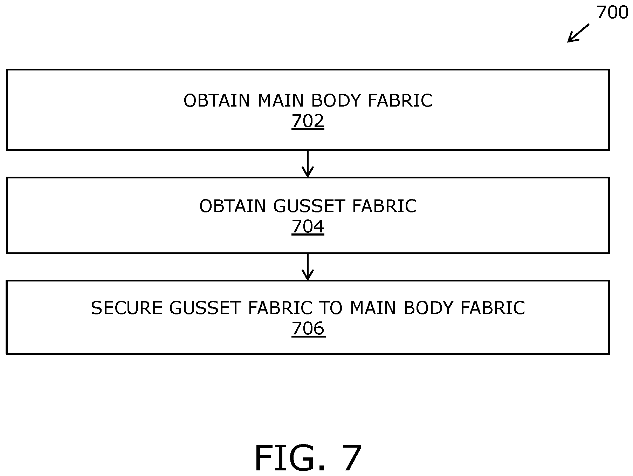

Referring to FIG. 7, illustrated is a flowchart **700** of steps of a method of preparing a lower body garment (such as the lower body garment **100, 200**, **300**, **400**, **500**, **600**) for a woman, in accordance with an aspect of the disclosed embodiments. At step **702**, a main body fabric **102, 202**, **302**, **402**, **502**, **602**, having an inner side **104,** an outer side **106** opposite the inner side **104,** a front end **108,** a rear end **110** opposite the front end **108,** a symmetrical vertical axis **A-A'** having a length **L1** between the front end **108** and the rear end **110,** and a pair of curved side ends **112, 114** each on either side of the symmetrical vertical axis **A-A'** having a width **W1** therebetween, is obtained.

At step **704**, a gusset fabric **116, 204**, **304**, **404**, **504**, **604**, having an inner side **118,** an outer side **120** opposite the inner side **118,** a front end **122,** a rear end **124** opposite the front end **122,** and a symmetrical vertical axis **B-B'** having a length **L2** between the front end **122** and the rear end **122,** and a pair of curved side ends **126, 128** each on either side of the symmetrical vertical axis **B-B'** having a width **W2** therebetween, is obtained.

At step **706**, the gusset fabric **116, 204**, **304**, **404**, **504**, **604** is secured to the main body fabric **102, 202**, **302**, **402**, **502**, **602** such that the outer side **120** of the gusset fabric **116, 204**, **304**, **404**, **504**, **604** is arranged to face the inner side **104** of the main body fabric **102, 202**, **302**, **402**, **502**, **602**, wherein the front end **122,** the rear end **124,** and the pair of curved side ends **126, 128** of the gusset fabric **116, 204**, **304**, **404**, **504**, **604** correspond to the main body fabric **102, 202**, **302**, **402**, **502**, **602**, and the front end **122,** the rear end **124,** and the pair of curved side ends **126, 128** of the gusset fabric **116, 204**, **304**, **404**, **504**, **604** are secured to the main body fabric **102, 202**, **302**, **402**, **502**, **602** at seams **130** of the gusset fabric **116, 204**, **304**, **404**, **504**, **604**, wherein the inner side **118** of the gusset fabric **116, 204**, **304**, **404**, **504**, **604** is operable to cover the vulva of the woman, when in use, and wherein the length **L2** corresponds to a length of the vulva.

The steps **702**, **704** and **706** are only illustrative and other alternatives can also be provided where one or more steps are added, one or more steps are removed, or one or more steps are provided in a different sequence without departing from the scope of the claims herein.

Various embodiments and variants disclosed above, with respect to the aforementioned system **(100, 200**, **300**, **400**, **500**, **600**), apply *mutatis mutandis* to the method. Beneficially, the method of the present disclosure ensures obtaining chemical-free fabrics for the gusset fabric **116, 204**, **304**, **404**, **504**, **604**, thereby preventing myriad gynaecological problems. Moreover, the design and securing of the gusset fabric **116, 204**, **304**, **404**, **504**, **604** to the main body fabric **102, 202**, **302**, **402**, **502**, **602** provides full coverage of the female genitalia to enhance female health and hygiene, while still providing a robust product that does not shift or move while in use or washing. Additionally, beneficially, the methods of securing the gusset fabric **116, 204**, **304**, **404**, **504**, **604** to the main body fabric **102, 202**, **302**, **402**, **502**, **602** ensures a more secure garment resulting in better comfort and fit experience to the user of the different styles of lower body garment **100, 200**, **300**, **400**, **500**, **600.**

Optionally, the gusset fabric **116, 204**, **304**, **404**, **504**, **604** and the main body fabric **102, 202**, **302**, **402**, **502**, **602** are secured using at least one of: a stitch, a bond, an adhesive.

Optionally, the gusset fabric **116, 204**, **304**, **404**, **504**, **604** is secured to the main body fabric **102, 202**, **302**, **402**, **502**, **602** along at least one of: the front end **122** of the gusset fabric **116, 204**, **304**, **404**, **504**, **604**, the rear end **124** of the gusset fabric **116, 204**, **304**, **404**, **504**, **604**, the pair of curved side ends **126, 128** of the gusset fabric **116, 204**, **304**, **404**, **504**, **604**, or a combination thereof.

Optionally, the gusset fabric **116, 204**, **304**, **404**, **504**, **604** is bonded on the inner side **118** thereof that covers the vulva.

Optionally, the main body fabric **102, 202**, **302**, **402**, **502**, **602** is selected from a group comprising: organic cotton, cotton, cotton spandex, bamboo, bamboo spandex, hemp, linen, muslin, synthetic, wool, wool blend, elastane, rayon, nylon, satin, silk, lace, organza, jersey, net, charmeuse, lycra, spandex, polyester, micro modal, merino wool, or any combination thereof.

Optionally, the gusset fabric **116, 204**, **304**, **404**, **504**, **604** is selected from organic cotton, cotton, linen, muslin, bamboo, hemp, wool, wool blend, elastane, or any other fabric combination.

Optionally, the length **L2** is smaller than the length **L1** and is at least 5 inches and curved at the front end **122** of the gusset fabric **116, 204**, **304**, **404**, **504**, **604** to cover the vulva.

Optionally, the widths **W1** and **W2** are equal.

Modifications to embodiments of the present disclosure described in the foregoing are possible without departing from the scope of the present disclosure as defined by the accompanying claims.

## Claims

1. A lower body garment for a woman 100, the garment comprising:
- a main body fabric (102) having an inner side (104), an outer side (106) opposite the inner side (104), a front end (108), a rear end (110) opposite the front end (108), a symmetrical vertical axis A-A' having a length L1 between the front end (108) and the rear end (110), and a pair of curved side ends (112, 114) each on either side of the symmetrical vertical axis A-A' having a width W1 therebetween; and
- a gusset fabric (116) having an inner side (118), an outer side (120) opposite the inner side (118), a front end (122), a rear end (124) opposite the front end (122), a symmetrical vertical axis B-B' having a length L2 between the front end (122) and the rear end (124), and a pair of curved side ends (126, 128) each on either side of the symmetrical vertical axis B-B' having a width W2 therebetween, wherein the length L2 is smaller than the length L1 and is at least 12,7 cm and wherein the front end (122) of the gusset fabric (116) is curved and configured to cover a vulva of the woman, and wherein the width W2 is +/- 0.13-0.38 cm than the width W1, the gusset fabric (116) secured to the main body fabric (102) such that:
- the outer side (120) of the gusset fabric (116) is arranged to face the inner side (104) of the main body fabric (102), wherein the front end (122), the rear end (124), and the pair of curved side ends (126, 128) of the gusset fabric (116) corresponds to the main body fabric (102), and
- the front end (122), the rear end (124), and the pair of curved side ends (126, 128) of the gusset fabric (116) are secured to the main body fabric (102) at seams (130) of the gusset fabric (116), wherein the gusset fabric (116) is secured to the main body fabric (102) along the front end (122) of the gusset fabric (116), the rear end (124) of the gusset fabric (116), the pair of curved side ends (126, 128) of the gusset fabric (116) and at a middle portion of the gusset fabric (116), wherein the middle portion corresponds to a portion between the pair of curved side ends (126, 128) of the gusset fabric (116) and the front end (122) of the gusset fabric (116) and the rear end (124) of the gusset fabric,
wherein the inner side (118) of the gusset fabric (116) is operable to cover the vulva of the woman, and wherein when in use, the length L2 corresponds to a length of the vulva.

2. The lower body garment according to claim 1, wherein the gusset fabric (116) is secured to the main body fabric (102) using at least one of: a stitch, a bond, an adhesive.

3. The lower body garment according to claim 1, wherein the gusset fabric (116) is bonded on the inner side (104) thereof that covers the vulva.

4. The lower body garment according to claim 1, wherein the main body fabric (102) is selected from a group comprising: organic cotton, cotton, cotton spandex, bamboo, bamboo spandex, hemp, linen, muslin, synthetic, wool, wool blend, elastane, rayon, nylon, satin, silk, lace, organza, jersey, net, charmeuse, LYCRA, spandex, polyester, micro modal, merino wool, or any combination thereof.

5. The lower body garment according to claim 1, wherein the gusset fabric (116) is selected from a group comprising: organic cotton, cotton, linen, muslin, bamboo, hemp, wool, wool blend, elastane, or any combination thereof.

6. A method for preparing a lower body garment for a woman, the method comprising:
- obtaining a main body fabric (102) having an inner side (104), an outer side (106) opposite the inner side (104), a front end (108), a rear end (110) opposite the front end (108), a symmetrical vertical axis A-A' having a length L1 between the front end (112, 114) and the rear end (110), and a pair of curved side ends (112, 114) each on either side of the symmetrical vertical axis A-A' having a width W1 therebetween;
- obtaining a gusset fabric (116) having an inner side (118), an outer side (120) opposite the inner side (118), a front end (122), a rear end (124) opposite the front end (122), and a symmetrical vertical axis B-B' having a length L2 between the front end (122) and the rear end (124), and a pair of curved side ends (126, 128) each on either side of the symmetrical vertical axis B-B' having a width W2 therebetween, wherein the length L2 is smaller than the length L1 and is at least 12,7 cm and wherein the front end (122) of the gusset fabric (116) is curved and configured to cover a vulva of the woman, and wherein the width W2 is +/-0.13-0.38 cm than the width W1; and
- securing the gusset fabric (116) to the main body fabric (102) such that:
- the outer side (120) of the gusset fabric (116) is arranged to face the inner side (104) of the main body fabric (102), wherein the front end (122), the rear end (124), and the pair of curved side ends (126, 128) of the gusset fabric (116) correspond to the main body fabric (102), and
- the front end (122), the rear end (124), and the pair of curved side ends (126, 128) of the gusset fabric (116) are secured to the main body fabric (102) at seams (130) of the gusset fabric (116), wherein the gusset fabric (116) is secured to the main body fabric (102) along the front end (122) of the gusset fabric (116), the rear end (124) of the gusset fabric (116), the pair of curved side ends (126, 128) of the gusset fabric (116) and at a middle portion of the gusset fabric, wherein the middle portion corresponds to a portion between the pair of curved side ends (126, 128) of the gusset fabric (116) and the front end (122) of the gusset fabric (116) and the rear end (124) of the gusset fabric (116),
wherein the inner side (118) of the gusset fabric (116) is operable to cover the vulva of the woman, and wherein when in use, the length L2 corresponds to a length of the vulva.

7. The method according to claim 6, wherein the gusset fabric (116) and the main body fabric (102) are secured using at least one of: a stitch, a bond, an adhesive.

8. The method according to claim 6, wherein the gusset fabric (116) is bonded on the inner side (104) thereof that covers the vulva.

9. The method according to claim 6, wherein the main body fabric (102) is selected from a group comprising: organic cotton, cotton, cotton spandex, bamboo, bamboo spandex, hemp, linen, muslin, synthetic, wool, wool blend, elastane, rayon, nylon, satin, silk, lace, organza, jersey, net, charmeuse, LYCRA, spandex, polyester, micro modal, merino wool, or any combination thereof.

10. The method according to claim 6, wherein the gusset fabric (116) is selected from a group comprising: organic cotton, cotton, linen, muslin, bamboo, hemp, wool, wool blend, elastane or any other fabric combination.

## Patentansprüche

1. Kleidungsstück für den Unterkörper für eine Frau (100), wobei das Kleidungsstück Folgendes umfasst:
- einen Hauptkörperstoff (102) mit einer Innenseite (104), einer Außenseite (106) gegenüber der Innenseite (104), einem vorderen Ende (108), einem hinteren Ende (110) gegenüber dem vorderen Ende (108), einer symmetrischen vertikalen Achse A-A' mit einer Länge L1 zwischen dem vorderen Ende (108) und dem hinteren Ende (110) und einem Paar bogenförmiger Seitenenden (112, 114) jeweils auf jeder Seite der symmetrischen vertikalen Achse A-A' mit einer Breite W1 dazwischen; und
- einen Zwickelstoff (116) mit einer Innenseite (118), einer Außenseite (120) gegenüber der Innenseite (118), einem vorderen Ende (122), einem hinteren Ende (124) gegenüber dem vorderen Ende (122), einer symmetrischen vertikalen Achse B-B' mit einer Länge L2 zwischen dem vorderen Ende (122) und dem hinteren Ende (124), und einem Paar bogenförmiger Seitenenden (126, 128) jeweils auf jeder Seite der symmetrischen vertikalen Achse B-B' mit einer Breite W2 dazwischen, wobei die Länge L2 kleiner ist als die Länge L1 und mindestens 12,7 cm beträgt
und wobei das vordere Ende (122) des Zwickelstoffs (116) bogenförmig und so ausgebildet ist, dass es eine Scham der Frau bedeckt, und wobei die Breite W2 +/- 0,13-0,38 cm im Verhältnis zu der Breite W1 beträgt, wobei der Zwickelstoff (116) derart an dem Hauptkörperstoff (102) befestigt ist, dass:
- die Außenseite (120) des Zwickelstoffs (116) so angeordnet ist, dass sie der Innenseite (104) des Hauptkörperstoffs (102) zugewandt ist, wobei das vordere Ende (122), das hintere Ende (124) und das Paar bogenförmiger Seitenenden (126, 128) des Zwickelstoffs (116) dem Hauptkörperstoff (102) entsprechen, und
- das vordere Ende (122), das hintere Ende (124) und das Paar bogenförmiger Seitenenden (126, 128) des Zwickelstoffs (116) an Nähten (130) des Zwickelstoffs (116) am Hauptkörperstoff (102) befestigt sind, wobei der Zwickelstoff (116) an dem Hauptkörperstoff (102) entlang dem vorderen Ende (122) des Zwickelstoffs (116), dem hinteren Ende (124) des Zwickelstoffs (116), dem Paar bogenförmiger Seitenenden (126, 128) des Zwickelstoffs (116) und an einem mittleren Abschnitt des Zwickelstoffs (116) befestigt ist, wobei der mittlere Abschnitt einem Abschnitt zwischen dem Paar bogenförmiger Seitenenden (126, 128) des Zwickelstoffs (116) und dem vorderen Ende (122) des Zwickelstoffs (116) und dem hinteren Ende (124) des Zwickelstoffs entspricht,
wobei die Innenseite (118) des Zwickelstoffs (116) dazu dient, die Scham der Frau zu bedecken, und wobei die Länge L2 im Gebrauch einer Länge der Scham entspricht.

2. Kleidungsstück für den Unterkörper nach Anspruch 1, wobei der Zwickelstoff (116) an dem Hauptkörperstoff (102) unter Verwendung von zumindest einem der Folgenden befestigt ist: einer Naht, einer Klebeverbindung, einem Haftmittel.

3. Kleidungsstück für den Unterkörper nach Anspruch 1, wobei der Zwickelstoff (116) an seiner Innenseite (104), die die Scham bedeckt, verbunden ist.

4. Kleidungsstück für den Unterkörper nach Anspruch 1, wobei der Hauptkörperstoff (102) ausgewählt ist aus einer Gruppe, bestehend aus: Bio-Baumwolle, Baumwolle, Baumwoll-Spandex, Bambus, Bambus-Spandex, Hanf, Leinen, Musselin, Synthetik, Wolle, Wollgemisch, Elastan, Viskose, Nylon, Satin, Seide, Spitze, Organza, Jersey, Netzgewebe, Charmeuse, LYCRA, Spandex, Polyester, Mikro-Modal, Merinowolle oder einer Kombination dieser.

5. Kleidungsstück für den Unterkörper nach Anspruch 1, wobei der Zwickelstoff (116) ausgewählt ist aus einer Gruppe, bestehend aus: Bio-Baumwolle, Baumwolle, Leinen, Musselin, Bambus, Hanf, Wolle, Wollgemisch, Elastan oder einer Kombination dieser.

6. Verfahren zur Herstellung eines Kleidungsstücks für den Unterkörper für eine Frau, wobei das Verfahren Folgendes umfasst:
Gewinnen eines Hauptkörperstoffs (102) mit einer Innenseite (104), einer Außenseite (106) gegenüber der Innenseite (104), einem vorderen Ende (108), einem hinteren Ende (110) gegenüber dem vorderen Ende (108), einer symmetrischen vertikalen Achse A-A' mit einer Länge L1 zwischen dem vorderen Ende (112, 114) und dem hinteren Ende (110) und einem Paar bogenförmiger Seitenenden (112, 114) jeweils auf jeder Seite der symmetrischen vertikalen Achse A-A' mit einer Breite W1 dazwischen;
Gewinnen eines Zwickelstoffs (116) mit einer Innenseite (118), einer Außenseite (120) gegenüber der Innenseite (118), einem vorderen Ende (122), einem hinteren Ende (124) gegenüber dem vorderen Ende (122) und einer symmetrischen vertikalen Achse B-B' mit einer Länge L2 zwischen dem vorderen Ende (122) und dem hinteren Ende (124) und einem Paar bogenförmiger Seitenenden (126, 128) jeweils auf jeder Seite der symmetrischen vertikalen Achse B-B' mit einer Breite W2 dazwischen, wobei die Länge L2 kleiner ist als die Länge L1 und zumindest 12,7 cm beträgt
und wobei das vordere Ende (122) des Zwickelstoffs (116) bogenförmig und so ausgebildet ist, dass es eine Scham der Frau bedeckt, und wobei die Breite W2 +/- 0,13-0,38 cm im Verhältnis zu der Breite W1 beträgt; und
Befestigen des Zwickelstoffs (116) an dem Hauptkörperstoff (102) derart, dass:
die Außenseite (120) des Zwickelstoffs (116) so angeordnet ist, dass sie der Innenseite (104) des Hauptkörperstoffs (102) zugewandt ist, wobei das vordere Ende (122), das hintere Ende (124) und das Paar bogenförmiger Seitenenden (126, 128) des Zwickelstoffs (116) dem Hauptkörperstoff (102) entsprechen und
das vorderen Ende (122), das hintere Ende (124), und das Paar bogenförmiger Seitenenden (126, 128) des Zwickelstoffs (116) an Nähten (130) des Zwickelstoffs (116) an dem Hauptkörperstoff (102) befestigt sind, wobei der Zwickelstoff (116) an dem Hauptkörperstoff (102) entlang dem vorderen Ende (122) des Zwickelstoffs (116), dem hinteren Ende (124) des Zwickelstoffs (116), dem Paar bogenförmiger Seitenenden (126, 128) des Zwickelstoffs (116) und an einem mittleren Abschnitt des Zwickelstoffs befestigt ist, wobei der mittlere Abschnitt einem Abschnitt zwischen dem Paar bogenförmiger Seitenenden (126, 128) des Zwickelstoffs (116) und dem vorderen Ende (122) des Zwickelstoffs (116) und dem hinteren Ende (124) des Zwickelstoffs (116) entspricht,
wobei die Innenseite (118) des Zwickelstoffs (116) dazu dient, die Scham der Frau zu bedecken, und wobei die Länge L2 im Gebrauch einer Länge der Scham entspricht.

7. Verfahren nach Anspruch 6, wobei der Zwickelstoff (116) und der Hauptkörperstoff (102) unter Verwendung von zumindest einem von einer Naht, einer Klebeverbindung und einem Haftmittel verbunden sind.

8. Verfahren nach Anspruch 6, wobei der Zwickelstoff (116) an seiner Innenseite (104), die die Scham bedeckt, verbunden ist.

9. Verfahren nach Anspruch 6, wobei der Hauptkörperstoff (102) ausgewählt ist aus einer Gruppe, bestehend aus: Bio-Baumwolle, Baumwolle, Baumwoll-Spandex, Bambus, Bambus-Spandex, Hanf, Leinen, Musselin, Synthetik, Wolle, Wollgemisch, Elastan, Viskose, Nylon, Satin, Seide, Spitze, Organza, Jersey, Netzgewebe, Charmeuse, LYCRA, Spandex, Polyester, Mikro-Modal, Merinowolle oder einer Kombination dieser.

10. Verfahren nach Anspruch 6, wobei der Zwickelstoff (116) ausgewählt ist aus einer Gruppe, bestehend aus: Bio-Baumwolle, Baumwolle, Leinen, Musselin, Bambus, Hanf, Wolle, Wollgemisch, Elastan oder einer anderen Stoffkombination.

## Revendications

1. Vêtement de bas du corps pour femme (100), le vêtement comprenant :
- un tissu de corps principal (102) ayant un côté interne (104), un côté externe (106) opposé au côté interne (104), une extrémité avant (108), une extrémité arrière (110) opposée à l'extrémité avant (108), un axe vertical symétrique A-A' ayant une longueur L1 entre l'extrémité avant (108) et l'extrémité arrière (110), et une paire d'extrémités latérales incurvées (112, 114) chacune de chaque côté de l'axe vertical symétrique A-A' ayant une largeur W1 entre elles ; et
- un tissu de gousset (116) ayant un côté interne (118), un côté externe (120) opposé au côté interne (118), une extrémité avant (122), une extrémité arrière (124) opposée à l'extrémité avant (122), un axe vertical symétrique B-B' ayant une longueur L2 entre l'extrémité avant (122) et l'extrémité arrière (124), et une paire d'extrémités latérales incurvées (126, 128) chacune de chaque côté de l'axe vertical symétrique B-B' ayant une largeur W2 entre elles, dans lequel la longueur L2 est plus petite que la longueur L1 et est d'au moins 12,7 cm
et dans lequel l'extrémité avant (122) du tissu de gousset (116) est incurvée et configurée pour couvrir une vulve de la femme, et dans lequel la largeur W2 est de +/- 0,13 à 0,38 cm par rapport à la largeur W1, le tissu de gousset (116) étant fixé au tissu de corps principal (102) de telle sorte que :
- le côté externe (120) du tissu de gousset (116) est agencé pour faire face au côté interne (104) du tissu de corps principal (102), dans lequel l'extrémité avant (122), l'extrémité arrière (124) et la paire d'extrémités latérales incurvées (126, 128) du tissu de gousset (116) correspondent au tissu de corps principal (102), et
- l'extrémité avant (122), l'extrémité arrière (124) et la paire d'extrémités latérales incurvées (126, 128) du tissu de gousset (116) sont fixées au tissu de corps principal (102) au niveau de coutures (130) du tissu de gousset (116), dans lequel le tissu de gousset (116) est fixé au tissu de corps principal (102) le long de l'extrémité avant (122) du tissu de gousset (116), de l'extrémité arrière (124) du tissu de gousset (116), de la paire d'extrémités latérales incurvées (126, 128) du tissu de gousset (116) et au niveau d'une portion médiane du tissu de gousset (116), dans lequel la portion médiane correspond à une portion entre la paire d'extrémités latérales incurvées (126, 128) du tissu de gousset (116) et l'extrémité avant (122) du tissu de gousset (116) et l'extrémité arrière (124) du tissu de gousset,
dans lequel le côté interne (118) du tissu de gousset (116) est utilisable pour couvrir la vulve de la femme, et dans lequel, en utilisation, la longueur L2 correspond à une longueur de la vulve.

2. Vêtement de bas du corps selon la revendication 1, dans lequel le tissu de gousset (116) est fixé au tissu de corps principal (102) à l'aide d'au moins l'un parmi : un point de couture, une liaison, un adhésif.

3. Vêtement de bas du corps selon la revendication 1, dans lequel le tissu de gousset (116) est lié sur son côté interne (104) qui couvre la vulve.

4. Vêtement de bas du corps selon la revendication 1, dans lequel le tissu de corps principal (102) est choisi dans un groupe comprenant : le coton biologique, le coton, le coton spandex, le bambou, le bambou spandex, le chanvre, le lin, la mousseline, le synthétique, la laine, la laine mélangée, l'élasthanne, la rayonne, le nylon, le satin, la soie, la dentelle, l'organza, le jersey, le filet, la charmeuse, le LYCRA, le spandex, le polyester, le micromodal, la laine mérinos, ou toute combinaison de ceux-ci.

5. Vêtement de bas du corps selon la revendication 1, dans lequel le tissu de gousset (116) est choisi dans un groupe comprenant : le coton biologique, le coton, le lin, la mousseline, le bambou, le chanvre, la laine, la laine mélangée, l'élasthanne, ou toute combinaison de ceux-ci.

6. Procédé de préparation d'un vêtement de bas du corps pour femme, le procédé comprenant :
l'obtention d'un tissu de corps principal (102) ayant un côté interne (104), un côté externe (106) opposé au côté interne (104), une extrémité avant (108), une extrémité arrière (110) opposée à l'extrémité avant (108), un axe vertical symétrique A-A' ayant une longueur L1 entre l'extrémité avant (112, 114) et l'extrémité arrière (110), et une paire d'extrémités latérales incurvées (112, 114) chacune de chaque côté de l'axe vertical symétrique A-A' ayant une largeur W1 entre elles ;
l'obtention d'un tissu de gousset (116) ayant un côté interne (118), un côté externe (120) opposé au côté interne (118), une extrémité avant (122), une extrémité arrière (124) opposée à l'extrémité avant (122), un axe vertical symétrique B-B' ayant une longueur L2 entre l'extrémité avant (122) et l'extrémité arrière (124), et une paire d'extrémités latérales incurvées (126, 128) chacune de chaque côté de l'axe vertical symétrique B-B' ayant une largeur W2 entre elles, dans lequel la longueur L2 est plus petite que la longueur L1 est d'au moins 12,7 cm
et dans lequel l'extrémité avant (122) du tissu de gousset (116) est incurvée et configurée pour couvrir une vulve de la femme, et dans lequel la largeur W2 est de +/- 0,13 à 0,38 cm par rapport à la largeur W1 ; et
la fixation du tissu de gousset (116) au tissu de corps principal (102) de sorte que :
le côté externe (120) du tissu de gousset (116) est agencé pour faire face au côté interne (104) du tissu de corps principal (102), dans lequel l'extrémité avant (122), l'extrémité arrière (124) et la paire d'extrémités latérales incurvées (126, 128) du tissu de gousset (116) correspondent au tissu de corps principal (102), et
l'extrémité avant (122), l'extrémité arrière (124), et la paire d'extrémités latérales incurvées (126, 128) du tissu de gousset (116) sont fixées au tissu de corps principal (102) au niveau de coutures (130) du tissu de gousset (116), dans lequel le tissu de gousset (116) est fixé au tissu de corps principal (102) le long de l'extrémité avant (122) du tissu de gousset (116), de l'extrémité arrière (124) du tissu de gousset (116), de la paire d'extrémités latérales incurvées (126, 128) du tissu de gousset (116) et au niveau d'une portion médiane du tissu de gousset (116), dans lequel la portion médiane correspond à une portion entre la paire d'extrémités latérales incurvées (126, 128) du tissu de gousset (116) et l'extrémité avant (122) du tissu de gousset (116) et l'extrémité arrière (124) du tissu de gousset,
dans lequel le côté interne (118) du tissu de gousset (116) est utilisable pour couvrir la vulve de la femme, et dans lequel, en utilisation, la longueur L2 correspond à une longueur de la vulve.

7. Procédé selon la revendication 6, dans lequel le tissu de gousset (116) et le tissu de corps principal (102) sont fixés à l'aide d'au moins l'un parmi : un point de couture, une liaison, un adhésif.

8. Procédé selon la revendication 6, dans lequel le tissu de gousset (116) est lié sur son côté interne (104) qui couvre la vulve.

9. Procédé selon la revendication 6, dans lequel le tissu de corps principal (102) est choisi dans un groupe comprenant : le coton biologique, le coton, le coton spandex, le bambou, le bambou spandex, le chanvre, le lin, la mousseline, le synthétique, la laine, la laine mélangée, l'élasthanne, la rayonne, le nylon, le satin, la soie, la dentelle, l'organza, le jersey, le filet, la charmeuse, le LYCRA, le spandex, le polyester, le micromodal, la laine mérinos, ou toute combinaison de ceux-ci.

10. Procédé selon la revendication 6, dans lequel le tissu de gousset (116) est choisi dans un groupe comprenant : le coton biologique, le coton, le lin, la mousseline, le bambou, le chanvre, la laine, la laine mélangée, l'élasthanne, ou toute combinaison de ceux-ci.
